(19) Europäisches Patentamt
European Patent Office
Office européen des brevets

(11) **EP 3 410 441 A1**

(12) **EUROPEAN PATENT APPLICATION**

(43) Date of publication:
**05.12.2018 Bulletin 2018/49**

(51) Int Cl.:
*G16H 30/40* (2018.01)    *G16H 50/70* (2018.01)

(21) Application number: **18175382.3**

(22) Date of filing: **31.05.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.05.2017  JP 2017108237**

(71) Applicant: **CANON KABUSHIKI KAISHA Ohta-ku Tokyo 146-8501 (JP)**

(72) Inventor: **KAWAGISHI, Masami Ohta-ku, Tokyo 146-8501 (JP)**

(74) Representative: **Houle, Timothy James Canon Europe Ltd European Patent Department 3 The Square Stockley Park Uxbridge, Middlesex UB11 1ET (GB)**

(54) **INFORMATION PROCESSING APPARATUS, INFORMATION PROCESSING METHOD, INFORMATION PROCESSING SYSTEM, AND PROGRAM**

(57)    An information processing apparatus acquires medical information about a target case, acquires a diagnostic name of the target case to be inferred based on the medical information, acquires an influence rate representing a degree of an influence of the medical information on the inference of the diagnostic name, and acquires a similar case as a case similar to the target case based on the medical information and the influence rate.

FIG. 4

**Description**

BACKGROUND

Field of the Invention

The present invention relates to an information processing apparatus, an information processing method, an information processing system, and a program.

Description of the Related Art

A system in which a computer analyzes a medical image and provides similar cases for assisting doctor's radiogram interpretation is proposed. Japanese Patent Application Laid-Open No. 2011-118543 discusses that, based on a feature amount extracted from a diagnostic target image, a similar image similar to the diagnostic target image is retrieved and is presented.

SUMMARY

According to a first aspect of the present invention, there is provided an information processing apparatus as specified in claims 1 to 19. According to a second aspect of the present invention, there is provided a method for controlling an information processing apparatus as specified in claim 20. According to a third aspect of the present invention, there is provided a program for controlling the information processing apparatus as specified in claim 21.
Further features of the present invention will become apparent from the following description of exemplary embodiments with reference to the attached drawings.

BRIEF DESCRIPTION OF THE DRAWINGS

Fig. 1 is a block diagram illustrating an example of a functional configuration of an information processing apparatus according to an exemplary embodiment.
Fig. 2 is a block diagram illustrating an example of a hardware configuration of the information processing apparatus according to the exemplary embodiment.
Fig. 3 is a flowchart illustrating an example of processing to be executed by the information processing apparatus according to the exemplary embodiment.
Fig. 4 is a diagram illustrating an example of a screen displayed by the information processing apparatus according to the exemplary embodiment.
Fig. 5 is a diagram illustrating an example of a screen displayed by the information processing apparatus according to the exemplary embodiment.
Fig. 6 is a diagram illustrating an example of a screen displayed by the information processing apparatus according to the exemplary embodiment.

DESCRIPTION OF THE EMBODIMENTS

There arises such a problem that information to be a clue for guiding a doctor to get a diagnostic name through observation of a diagnostic target image may not be obtained only by searching for a similar image based on the diagnostic target image and a feature amount. Exemplary embodiments for solving this problem will be described below with reference to the drawings.
An information processing apparatus 100 according to a first exemplary embodiment infers a diagnostic name based on medical information (including medical images) about a target case, and searches for cases having information to be clues for deriving a diagnostic name from the target case. Specifically, the information processing apparatus 100 searches for cases that are similar to the target case in a feature to be a positive clue to a diagnostic name derived from the target case but is not similar in a feature to be a negative clue to the diagnostic name.
The following description refers to a case where a target case is a case relating to a chest and a chest X-ray computed tomography (CT) image is used as a medical image of the target case. The chest X-ray CT image is supplied to a doctor as, for example, a medical image relating to radiogram interpretation of an abnormal shadow on a lung. Needless to say, the target is not limited to this case, and thus all diagnostic names and medical information described below are only examples for describing processing to be executed by the information processing apparatus 100.

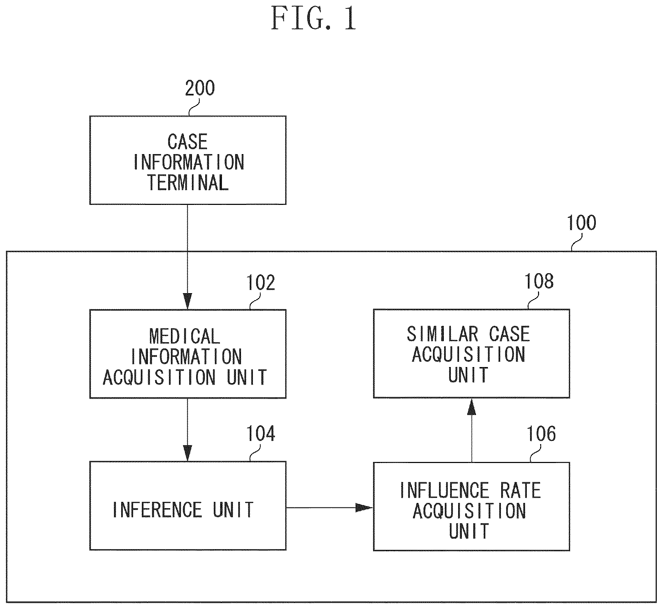

Fig. 1 is a diagram illustrating an example of a functional configuration of the information processing apparatus 100. The information processing apparatus 100 is connected to a case information terminal 200. The case information terminal 200 acquires medical information about a case to be diagnosed (hereinafter, referred to as a target case) from a server (not illustrated) such as a picture archiving and communication system or an external storage device such as a hard disk drive (HDD) or a digital versatile disk (DVD) drive. The case information terminal 200 transmits the medical information to the information processing apparatus 100 via a local area network (LAN) or the like. The medical information is information to be acquired in a diagnosis relating to the target case, and includes a medical image, information associated with the medical image, and clinical information. The information associated with a medical image includes an image feature amount acquired by executing image processing on a medical image and information about findings representing the features of the medical image. The information about findings is words and numerical values representing the features of the medical image. The information about findings may be recorded in form of items and values of the items. The values of the items of findings in this case are, for example, words and numerical values representing the features of the medical image. The information about findings may be input in the medical image by a user such as a doctor through the case information terminal 200. The case information terminal 200 may acquire the information about findings by analyzing the medical image. The case information terminal 200 may acquire the information about findings input or analyzed and acquired in an external device (not illustrated). The medical image has a form based on, for example, digital imaging and communications in medicine (DICOM). The information associated with the medical image includes various pieces of information defined by the DICOM. The clinical information is information acquired by a doctor during examination of a patient in the target case. The clinical information may include information representing results of various kinds of tests such as a blood test. The case information terminal 200 may acquire the clinical information from information described in an electronic health record.

The information processing apparatus 100 includes a medical information acquisition unit 102, an inference unit 104, an influence rate acquisition unit 106, and a similar case acquisition unit 108.

The medical information acquisition unit 102 acquires medical information about a target case from the case information terminal 200. The medical information acquisition unit 102 is an example of an information acquisition unit.

The inference unit 104 executes an inference using the acquired medical information, and derives a diagnostic name of the target case. The inference unit 104 is an example of an inference name acquit ion unit.

The influence rate acquisition unit 106 acquires influence rates that are degrees of influences to be exerted on the diagnostic name by the respective pieces of medical information. The influence rate acquisition unit 106 is an example of an influence rate acquisition unit.

The similar case acquisition unit 108 sets weights based on the influence rates to make a searching to acquire similar cases. The similar case acquisition unit 108 searches a medical case database (hereinafter, referred to as case DB, not illustrated) to acquire similar cases. The similar case acquisition unit 108 is an example of a similar case acquisition unit.

The case DB (not illustrated) may be provided in the information processing apparatus 100 or may be disposed outside the information processing apparatus 100. The similar case acquisition unit 108 may be connected to the case DB via the case information terminal 200. In such a case, the case DB may be provided in the case information terminal 200. The case DB (not illustrated) stores information about a plurality of cases and information associated with the cases. The information associated with the cases includes diagnostic names related to the cases and information about findings representing features of medical images of the cases. The case DB (not illustrated) may further store statistical information and document information such as books on medical science and medical papers.

At least one of the respective units of the information processing apparatus 100 illustrated in Fig. 1 may be realized as an independent device. Further, the respective units may be realized as software that realizes functions of the units. In the first exemplary embodiment, the respective functional configurations are realized by software.

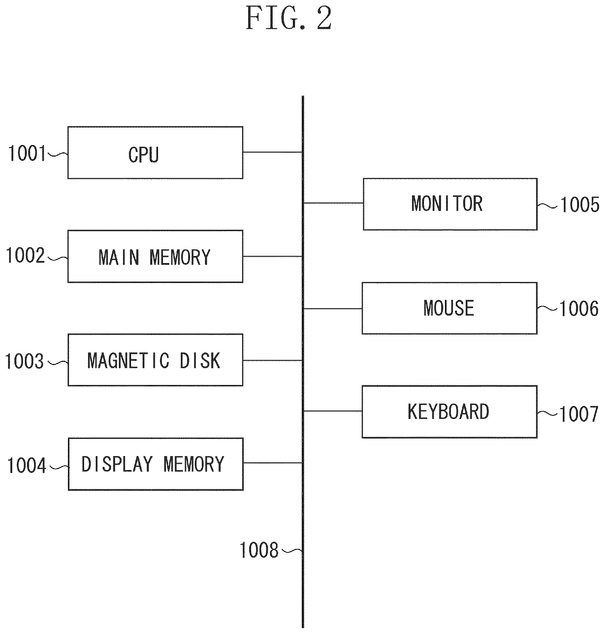

Fig. 2 is a block diagram illustrating an example of a hardware configuration of the information processing apparatus 100. A central processing unit (CPU) 1001 mainly controls operations of the components. A main memory 1002 stores control programs to be executed by the CPU 1001, and provides a work area used when the CPU 1001 executes the programs. A magnetic disk 1003 stores an operating system (OS), device drivers for peripheral devices, and programs for realizing various application software including programs for executing processing, described below. The CPU 1001 executes the programs stored in the main memory 1002 and the magnetic disk 1003 to realize the functions (software) of the information processing apparatus 100 illustrated in Fig. 1 and processing in a flowchart, described below.

A display memory 1004 temporarily stores display data. A monitor 1005 is, for example, a cathode ray tube (CRT) monitor or a liquid crystal monitor. The monitor 1005 displays images and texts based on data from the display memory 1004. A mouse 1006 and a keyboard 1007 are used by a user to perform pointing input and input of characters, respectively. The above-described respective components are communicably connected to each other by a common bus 1008.

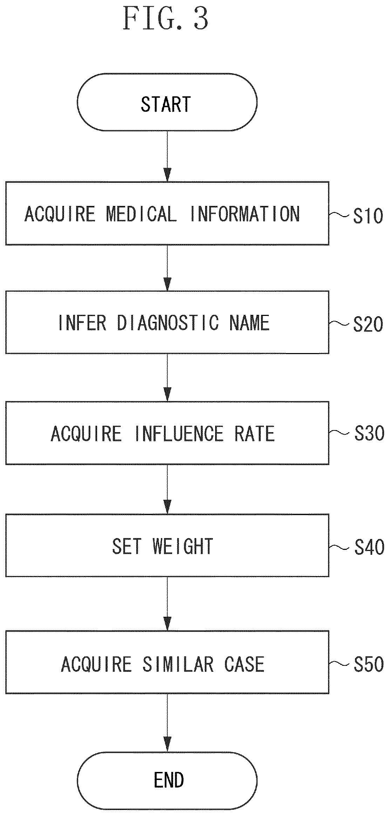

Fig. 3 is a flowchart illustrating an example of processing to be executed by the information processing apparatus 100. In the first exemplary embodiment, the CPU 1001 executes the programs that are stored in the main memory 1002 and realize the functions of the respective units. As a result, the processing illustrated in Fig. 3 is realized.

In the following descriptions, each medical information is represented by $E_k$, a value of the medical information is represented by $e_k$, and a set of the values $e_k$ (i.e., input to the inference unit 104) is represented by E. In a case where the medical information is information about findings, in the first exemplary embodiment, all the values $e_k$ of $E_k$ have discrete values. The medical information $E_k$ corresponds to items of the findings, and the value $e_k$ corresponds to values of the items of the findings. For example, in a case where the medical information $E_k$ is image findings "spicula", the value $e_k$ has any one of four discrete values "strong", "intermediate", "weak", and "none". In a case where the medical information $E_k$ is clinical information "the number of white blood cells", the value $e_k$ has any one of two discrete values "normal range" and "abnormal range". The respective values of the medical information may be any values of continuous values.

In step S10, the medical information acquisition unit 102 acquires medical information transmitted from the case information terminal 200. The medical information includes image findings of abnormal shadow on a lung, an image feature amount acquired by executing image processing on the abnormal shadow, and clinical information. The first exemplary embodiment will be described for, as an example, a case where information about findings (hereinafter, referred to as image findings) input into a medical image by a user and clinical information are acquired as the medical information.

In step S20, the inference unit 104 infers a diagnostic name of a target case using the medical information acquired in step S10. In the inference, existing various methods (inference methods) such as a Bayesian network, a support vector machine, and a neural network can be used. In the first exemplary embodiment, the Bayesian network is used.

In step S30, the influence rate acquisition unit 106 acquires influence rates of the medical information acquired in step S10 with respect to the diagnostic name inferred in step S20. The influence rates are acquired by using, for example, an expression 1.

$$I(E_k) = P(D|E) - P(D|E - e_k) \cdots (\text{Expression 1})$$

In the expression 1, D represents an inferred diagnostic name, $I(E_k)$ represents an influence rate of the medical information $E_k$ with respect to the diagnostic name D, and P(D|E) represents posterior probability of the diagnostic name D in a case where the value set E is input. In a case where the value in the expression 1 is a positive value, if the value $e_k$ of the medical information $E_k$ is subtracted from the value set E, the posterior probability of the diagnostic name D reduces. For this reason, $E_k$ represents medical information exerting positive influence on the inference of the diagnostic name D. In a case where the value in the expression 1 is a negative value, if the value $e_k$ of the medical information $E_k$ is subtracted from the value set E, the posterior probability of the diagnostic name D increases. Therefore, $E_k$ represents medical information that exerts a negative influence on the inference of the diagnostic name. Needless to say, the above-described method for acquiring an influence rate is only an example, and another method may be used. For example, an influence rate may be acquired by using an expression 2.

$$I(Ek) = P(D|e_k) - P(D) \cdots (\text{Expression 2})$$

In the expression 2, P(D) represents prior probability of the diagnostic name D. In a case where a value in the expression 2 is a positive value, if the value $e_k$ of the medical information $E_k$ is input, the posterior probability of the diagnostic name D increases. Therefore, similar to the expression 1, the medical information $E_k$ is information that exerts a positive influence on the inference of the diagnostic name D. On the other hand, in a case where a value in the expression 2 is a negative value, if the value $e_k$ of the medical information $E_k$ is input, the posterior probability of the diagnostic name D decreases. Therefore, similar to the expression 1, the medical information $E_k$ is information that exerts a negative influence on the inference of the diagnostic name D.

In this way, the influence rate acquisition unit 106 acquires the influence rates discriminably for the case where medical information exerts a positive influence on the inference of a diagnostic name and the case where medical information exerts a negative influence on the inference of a diagnostic name.

In step S40, the similar case acquisition unit 108 sets weights of the respective pieces of medical information based on the influence rates, acquired in step S30, on the diagnostic name of the medical information. In the first exemplary embodiment, as a weight $W_k$ for the medical information $E_k$, the influence rate $I(E_k)$ of the medical information $E_k$ with respect to a diagnostic name is used.

In step S50, the similar case acquisition unit 108 searches the case DB (not illustrated) for similar cases of the target case, based on the plural pieces of medical information acquired in step S10 and the weights set in step S40 for the medical information.

The similar cases are searched for based on, for example, an expression 3. According to the expression 3, cases with higher similarity to be acquired are retrieved as more similar cases.

$$\text{Sim}(C_j) = \Sigma(W_k \cdot f(e_k, e_{kj})) \cdots (\text{Expression 3})$$

In the expression 3, $C_j$ represents a retrieved case, $\text{Sim}(C_j)$ represents similarity between a target case and the retrieved case $C_j$, $e_{kj}$ represents a value of the medical information $E_k$ in the retrieved case $C_j$, and $f(e_k, e_{kj})$ represents a function where $e_k$ and $e_{kj}$ are inputs. In the first exemplary embodiment, the function $f(e_k, e_{kj})$ has 1 if the values $e_k$ and $e_{kj}$ agree with each other, and has 0 if the values do not agree.

In the first exemplary embodiment, the similarity is a sum of the weights of medical information with values that coincide with each other between the target case and the retrieved cases (i.e., the influence rates). In other words, the similarity becomes higher as the values of positive information have better agreement between the target case and the retrieved cases (the influence rates have a positive value), and the similarity becomes lower as the values of negative information have better agreement (the influence rates have a negative value). As a result, such retrieved cases that positive information has similarity and negative information has non-similarity, are retrieved as the similar cases.

The above-described similarity acquisition method is only one example, and thus another method may be used. For example, if the values $e_k$ and $e_{kj}$ have continuous values, the function $f(e_k, e_{kj})$ may have an absolute value of a difference between the two values.

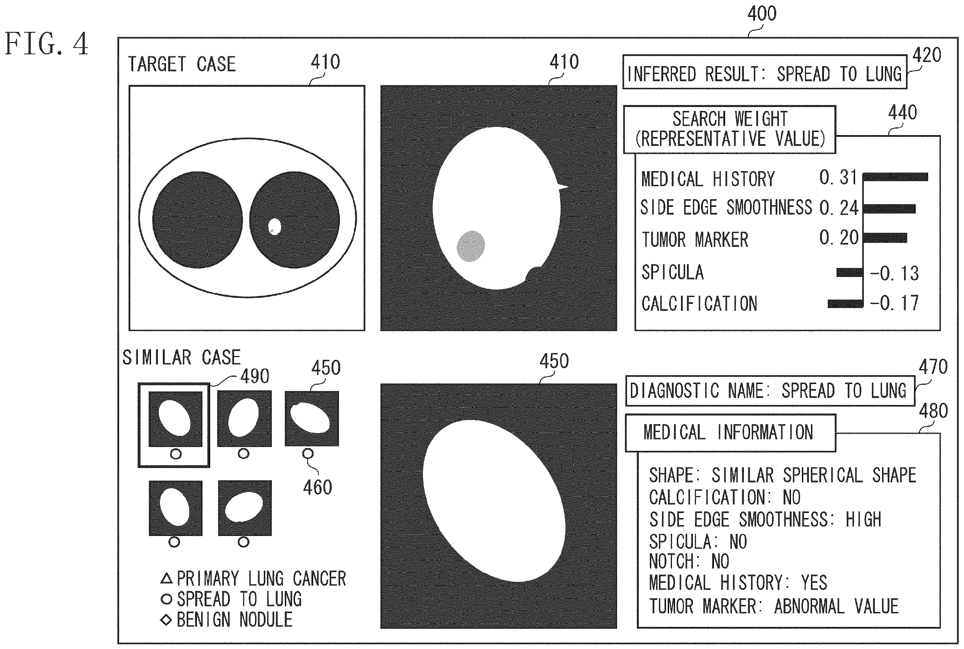

The similar case acquisition unit 108 provides information for enabling a comparison between the acquired similar cases and the target case to a user such as a doctor. The similar case acquisition unit 108 may display the information on a display unit (not illustrated) such as a monitor connected to the information processing apparatus 100 or a display unit included in the case information terminal 200. For example, the similar case acquisition unit 108 displays a graphical user interface (GUI) 400 illustrated in Fig. 4 on the display unit (not illustrated). From this aspect, the similar case acquisition unit 108 is an example of a display control unit. A doctor checks similar cases in which an inferred result is reflected so as to be able to use the similar cases as reference information for diagnosis.

Fig. 4 is a diagram illustrating an example of a screen displayed by the information processing apparatus 100. The GUI 400 includes a target case 410, an inferred result 420, a search weight 440, and a similar case 450. The GUI 400 further includes a diagnostic name icon 460 of a similar case, diagnostic name information 470 of the similar case, and medical information 480 of the similar case.

The target case 410 is an example of information relating to a target case. For example, a medical image included in the medical information acquired in step S10 is displayed as the target case 410. The inferred result 420 indicates the diagnostic name inferred in step S20. The search weight 440 is the weight set in step S40. In Fig. 9, a weight of medical information with a large absolute value of the weight is displayed as a representative value on the search weight 440. Weights set for all pieces of medical information may be displayed on the search weight 440, or some of them may be displayed. Alternatively, the display may be switched between all the weights and some of the weights.

The similar case 450 is an example of information relating to the similar cases. A case that is included in the similar cases retrieved in step S50 and is selectively specified by a user (a case surrounded by a rectangle 490) is displayed as the similar case 450. Furthermore, in the example illustrated in Fig. 4, the information about diagnostic names of the similar case 450 is displayed as the diagnostic name icon 460, and the diagnostic name information 470 and the medical information 480 about the selected similar case are displayed. The user can easily figure out diagnostic names of a similar case group in which an inferred result is reflected, by checking the diagnostic name icon 460. Further, the user can check details of the similar case and use these pieces of information as reference information for a diagnosis of the target case by referring to the diagnostic name information 470 and the medical information 480.

According to the first exemplary embodiment, the information processing apparatus 100 infers a diagnostic name from the medical information, acquires an influence rate of the medical information with respect to the inference, and searches for similar cases based on the acquired influence rate. As a result, the information processing apparatus 100 can easily retrieve similar cases including the medical information to be a clue for obtaining diagnostic names to be inferred from the target case.

<Modification Example>

In the first exemplary embodiment, in step S40, values of the influence rate have been used directly as the weights, but reciprocal numbers of the influence rates (i.e., -I(Ek)) may be used. Alternatively, in step S30, a negative influence rate may be acquired in a case of positive information, and a positive influence rate may be acquired in a case of negative information. In such a case, in step S40, the influence rate may be directly used as the weight. For example, by using an expression 4, the influence rate acquisition unit 106 can acquire a negative influence rate in a case of positive information, and can acquire a positive influence rate in a case of negative information.

$$I(E_k) = P(D|E -e_k) - P(D|E) \cdots (\text{Expression 4})$$

According to the expression 4, in step S50, the similarity becomes higher as values of negative information have better agreement, and the similarity becomes lower as values of positive information have better agreement. Accordingly, the similar case acquisition unit 108 retrieves and provides retrieved cases with negative information having similarity with respect to a target case and with positive information having non-similarity with respect to the target case as the similar cases. As a result, a user can check similar cases that are against the inferred result, and that can be used as reference information for a diagnosis of the target case.

The information processing apparatus 100 according to a second exemplary embodiment search for a similar case that is similar to a target case in information to be a positive clue to a diagnostic name to be inferred from the target case.

The information processing apparatus 100 according to the second exemplary embodiment has a functional configuration and a hardware configuration similar to those illustrated in Fig. 1 and Fig. 2. Detailed description thereof will be omitted by use of the above description.

Further, processing to be executed by the information processing apparatus 100 according to the second exemplary embodiment is illustrated in the flowchart of Fig. 3. The processing in step S10 to step S30 is similar to the processing in the first exemplary embodiment, and detailed description thereof is omitted by using the above description.

In step S40, the similar case acquisition unit 108 sets weights of the medical information based on the influence rates, acquired in step S30, on the diagnostic names of the medical information. In the second exemplary embodiment, if the influence rate $I(E_k)$ with respect to a diagnostic name of the medical information $E_k$ is positive, the influence rate $I(E_k)$ is set as a weight $W_k$ of the medical information $E_k$, if negative, $W_k = 0$.

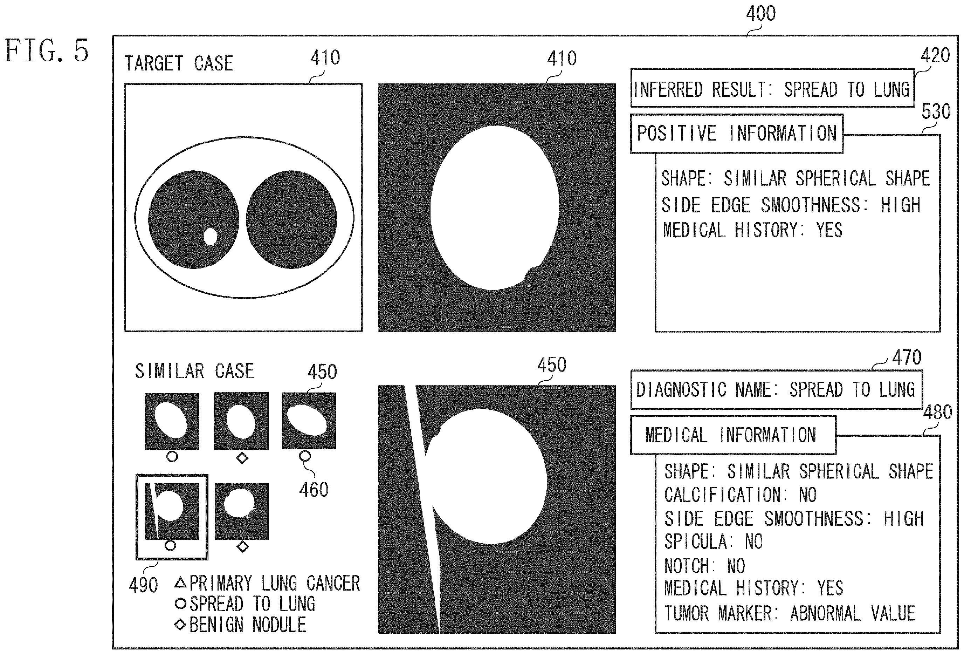

In step S50, the similar case acquisition unit 108 searches for a similar case of the target case based on the plurality of pieces of medical information acquired in step S10 and the weights of the respective pieces of medical information set in step S40. Mathematical expressions of the similarity are similar to the mathematical expressions in the first exemplary embodiment. Furthermore, the similar case acquisition unit 108 displays the GUI 400 illustrated in Fig. 5 on the display unit (not illustrated).

Fig. 5 is a diagram illustrating an example of a screen displayed by the information processing apparatus 10 according to the second exemplary embodiment. The GUI 400 includes the target case 410, the inferred result 420, positive information 530, and the similar case 450. The GUI 400 further includes the diagnostic name icon 460 of a similar case, the diagnostic name information 470 of the similar case, and the medical information 480 of the similar case. Description about components with reference numerals equal to the reference numerals of the components illustrated in Fig. 4 will be omitted below.

The positive information 530 indicates a part of medical information identified as information for affirming a diagnostic name based on the influence rates acquired in step S30. Information with larger influence rates is selectively displayed, but predetermined information may be displayed or all pieces of information may be displayed. Alternatively, the display may be switched between the predetermined information and all the pieces of information. A user can check information for affirming the inferred diagnostic name in accordance with the similar cases by checking the similar case 450, the positive information 530, and the medical information 480 about similar cases.

In the second exemplary embodiment, similarity is obtained by summing weights of positive medical information where values agree between a target case and retrieved cases. In this case, negative medical information is ignored. As a result, cases with only similarity of positive information are retrieved as the similar cases. Therefore, a doctor can check the information for affirming an inferred diagnostic name by using the similar cases, and can use the information as reference information for a diagnosis of the target case.

<Modification Example>

In the second exemplary embodiment, in step S40, if the influence rate has a positive value, the influence rate is used as a weight, and if the influence rate has a negative value, the weight is 0. However, the reverse case may be also allowable. More specifically, if the influence rate has a negative value, the weight may be a reciprocal number (i.e., -I(Ek)), and if the influence rate has a positive value, the weight may be 0. In this case, in step S50, weights of negative medical information where values agree between a target case and retrieved cases are summed, and positive medical information is ignored. As a result, cases with only similarity of negative information are retrieved as the similar cases. Therefore, a doctor can check the information for negating an inferred diagnostic name by using the similar cases, and can use the information as reference information for the diagnosis by the doctor.

The information processing apparatus 100 according to a third exemplary embodiment searches for similar cases with similarity of both information to be a positive clue to a diagnostic name to be inferred from a target case and information to be a negative clue to the diagnostic name.

The information processing apparatus 100 according to the third exemplary embodiment has the functional configuration and the hardware configuration similar to those illustrated in Fig. 1 and Fig. 2. Detailed description thereof will be omitted by using the above description.

Processing to be executed by the information processing apparatus 100 according to the third exemplary embodiment is illustrated in the flowchart of Fig. 3. Since the processing in step S10 to step S30 is similar to the processing in the first exemplary embodiment, detailed description thereof is omitted by using the above description.

In step S40, the similar case acquisition unit 108 sets weights of medical information based on the influence rates, acquired in step S30, on diagnostic names of medical information. In the third exemplary embodiment, an absolute value $|I(E_k)|$ of the influence rate $I(E_k)$ on a diagnostic name of the medical information $E_k$ is the weight $W_k$ of the medical information $E_k$.

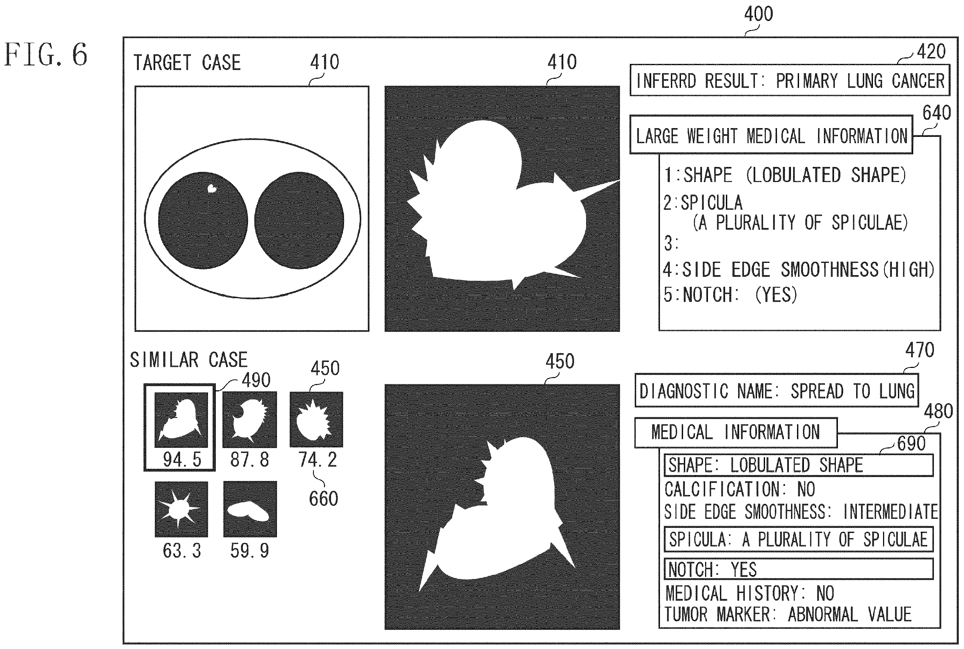

In step S50, the similar case acquisition unit 108 searches for similar cases of a target case based on the plurality of pieces of medical information acquired in step S10 and the weights of the medical information set in step S40. Mathematical expressions of the similarity are similar to the mathematical expressions in the first exemplary embodiment. Further, the similar case acquisition unit 108 displays the GUI 400 illustrated in Fig. 6 on the display unit (not illustrated).

Fig. 6 illustrates an example of a screen displayed by the information processing apparatus 100. The GUI 400 includes the target case 410, the inferred result 42, large-weight medical information 640, and the similar case 450. The GUI 400 further includes normalization similarity 660 of a similar case, the diagnostic name information 470 of the similar case, medical information 480 of the similar case, and an emphasis frame 690 of medical information. Description of the components with reference numerals equal to the reference numerals of the components in Fig. 4 will be omitted below.

The large-weight medical information 640 includes respective pieces of medical information that are displayed in decreasing order of the weights sets in step S40. Words within parentheses indicate values of the medical information in a target case. The normalization similarity 660 indicates a value obtained by normalizing the similarity calculated in step S50 into a value ranging from 0 to 100, and as the value is closer to 100, the similarity to the target case is higher. The emphasis frame 690 of large-weigh medical information is for emphasizing medical information in which values agree between a target case and a similar case. A user can check a similar case with similarity of information exerting a strong influence on an inferred diagnostic name by checking the similar case 450, the large-weight medical information 640, the normalization similarity 660, and the emphasis frame 690 of medical information. Further, the user can use the similar case as reference information for a diagnosis of a target case by checking the medical information 480 of the similar case and the emphasis frame 690 of the similar case.

In the third exemplary embodiment, since an absolute value of an influence rate is used as a weight, as agreement between values of positive information and negative information is higher, the similarity becomes higher. As a result, a case with similarity of both the positive information and the negative information is retrieved as a similar case. Therefore, a doctor can check the similar case with similarity of information exerting a strong influence on an inferred diagnostic name and can use the similar case as reference information for a diagnosis of a target case.

<Modification Example>

In the above-described exemplary embodiments, the description are given, as an example, of the case where values of findings are used as inputs and a diagnostic name is inferred, but the present invention is not limited thereto. For example, an image feature amount to be acquired by analyzing a medical image is compared with an image feature amount of a medical image of a case stored in the case DB, and a similar case may be obtained. The information processing apparatus 100 may only be required to be able to acquire information necessary for inference and retrieval of a similar case, and thus a medical image of a target case does not have to be necessarily acquired.

The information processing apparatus 100 may be configured to be able to select the similar case retrieval method from the methods in the above-described exemplary embodiments, in accordance with an instruction from a user.

The information processing apparatus according to the above-described exemplary embodiments may be realized as a single apparatus, or a plurality of apparatus may be communicably combined to execute the above-described processing. These forms are included in the exemplary embodiments of the present invention. A common server device or a server set may execute the above-described processing. A plurality of apparatuses included in the information processing apparatus and the information processing system may only be required to be communicable with each other at a predetermined communication rate. Further, the plurality of apparatuses does not have to be present in one facility or in one country.

Forms where the above-described exemplary embodiments are appropriately combined are also included in the exemplary embodiments of the present invention.

Other Embodiments

Embodiment (s) of the present invention can also be realized by a computer of a system or apparatus that reads out and executes computer executable instructions (e.g., one or more programs) recorded on a storage medium (which may also be referred to more fully as a 'non-transitory computer-readable storage medium') to perform the functions of one or more of the above-described embodiment(s) and/or that includes one or more circuits (e.g., application specific integrated circuit (ASIC)) for performing the functions of one or more of the above-described embodiment(s), and by a method performed by the computer of the system or apparatus by, for example, reading out and executing the computer executable instructions from the storage medium to perform the functions of one or more of the above-described embodiment(s) and/or controlling the one or more circuits to perform the functions of one or more of the above-described embodiment(s). The computer may comprise one or more processors (e.g., central processing unit (CPU), micro processing unit (MPU)) and may include a network of separate computers or separate processors to read out and execute the computer executable instructions. The computer executable instructions may be provided to the computer, for example, from a network or the storage medium. The storage medium may include, for example, one or more of a hard disk, a random-access memory (RAM), a read only memory (ROM), a storage of distributed computing systems, an optical disk (such as a compact disc (CD), digital versatile disc (DVD), or Blu-ray Disc (BD)™), a flash memory device, a memory card, and the like.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

**Claims**

1. An information processing apparatus comprising:

   information acquisition means for acquiring medical information about a target case;
   inferred name acquisition means for acquiring a diagnostic name to be interfered for the target case based on the medical information;
   influence rate acquisition means for acquiring an influence rate representing a degree of an influence of the medical information on the inference of the diagnostic name; and
   similar case acquisition means for acquiring a similar case as a case similar to the target case, based on the medical information and the influence rate.

2. The information processing apparatus according to claim 1, wherein the information acquisition means acquires information about a finding representing a feature of a medical image of the target case as the medical information about the target case.

3. The information processing apparatus according to claim 2, wherein the information about the finding representing the feature of the medical image of the target case is a value to be acquired for an item of the finding relating to the feature.

4. The information processing apparatus according to claim 2 or 3, wherein the information about the finding representing the feature of the medical image of the target case is a word representing the feature.

5. The information processing apparatus according to any one of claims 1 to 4, further comprising display control means for displaying information about the similar case on display means.

6. The information processing apparatus according to claim 5, wherein the display control means displays the information about the target case and the information about the similar case next to each other on the display means.

7. The information processing apparatus according to claim 6, wherein the display control means displays, as the information about the target case, the medical information that influences the inference of the diagnostic name.

8. The information processing apparatus according to any one of claim 5 to 7, wherein the display control means displays information about the influence rate as the information about the similar case, on the display means.

9. The information processing apparatus according to claim 8, wherein the display control means displays, on the display means, the information about the influence rate as information about a weight for searching to acquire the similar case.

10. The information processing apparatus according to any one of claims 5 to 9, wherein the display control means displays, on the display means, a medical image of the target case and a medical image of the similar case so that the medical images can be compared with each other.

11. The information processing apparatus according to any one of claims 1 to 10, wherein the influence rate acquisition means acquires the influence rate so that a case where the medical information exerts a positive influence on the inference of the diagnostic name can be distinguished from a case where the medical information exerts a negative influence on the inference of the diagnostic name.

12. The information processing apparatus according to any one of claims 1 to 11, wherein the influence rate acquisition means acquires the influence rate so that the influence rate has a positive value in a case where the medical information exerts a positive influence on the inference of the diagnostic name, and has a negative value in a case where the medical information exerts a negative influence on the inference of the diagnostic name.

13. The information processing apparatus according to any one of claims 1 to 11, wherein the influence rate acquisition means acquires the influence rate so that the influence rate has a negative value in a case where the medical information exerts a positive influence on the inference of the diagnostic name, and has a positive value in a case where the medical information exerts a negative influence on the inference of the diagnostic name.

14. The information processing apparatus according to any one of claims 1 to 13, wherein the similar case acquisition means searches the medical information and acquires the similar case by searching for the similar case using the acquired influence rate as a weight for the medical information.

15. The information processing apparatus according to claim 14, wherein the similar case acquisition means uses a reciprocal number of the influence rate as the weight.

16. The information processing apparatus according to claim 14, wherein the similar case acquisition means sets the weight to 0 in a case where the influence rate has a negative value.

17. The information processing apparatus according to claim 14, wherein the similar case acquisition means sets the weight to 0 in a case where the influence rate has a positive value.

18. The information processing apparatus according to claim 14, wherein the similar case acquisition means uses an absolute value of the influence rate as the weight.

19. The information processing apparatus according to any one of claims 5 to 10,
wherein the similar case acquisition means performs searching using the medical information, and performs searching using the acquired influence rate, as a weight on the medical information, and
wherein the display control means discriminably displays, on the display means, medical information that has a large weight and a value in agreement with a value of the medical information about the target case.

20. An information processing method comprising:

acquiring medical information about a target case;
acquiring a diagnostic name of the target case to be inferred based on the medical information;
acquiring an influence rate representing a degree of an influence of the medical information on the inference of the diagnostic name; and
acquiring a similar case as a case similar to the target case, based on the medical information and the influence rate.

21. A program for causing a computer to execute the information processing method of claim 20.

# FIG. 1

200

```
CASE
INFORMATION
TERMINAL
```

100

102

```
MEDICAL
INFORMATION
ACQUISITION UNIT
```

108

```
SIMILAR CASE
ACQUISITION
UNIT
```

104

```
INFERENCE UNIT
```

106

```
INFLUENCE RATE
ACQUISITION
UNIT
```

# FIG. 2

1001 — CPU

1002 — MAIN MEMORY

1003 — MAGNETIC DISK

1004 — DISPLAY MEMORY

1005 — MONITOR

1006 — MOUSE

1007 — KEYBOARD

1008

# FIG. 3

```
        ┌─────────────┐
        │    START    │
        └─────────────┘
               │
               ▼
┌──────────────────────────────┐
│  ACQUIRE MEDICAL INFORMATION  │ ～S10
└──────────────────────────────┘
               │
               ▼
┌──────────────────────────────┐
│      INFER DIAGNOSTIC NAME    │ ～S20
└──────────────────────────────┘
               │
               ▼
┌──────────────────────────────┐
│     ACQUIRE INFLUENCE RATE    │ ～S30
└──────────────────────────────┘
               │
               ▼
┌──────────────────────────────┐
│          SET WEIGHT          │ ～S40
└──────────────────────────────┘
               │
               ▼
┌──────────────────────────────┐
│     ACQUIRE SIMILAR CASE      │ ～S50
└──────────────────────────────┘
               │
               ▼
        ┌─────────────┐
        │     END     │
        └─────────────┘
```

FIG. 4

# FIG. 5

**TARGET CASE** 410     410     420

INFERRED RESULT: SPREAD TO LUNG

530

POSITIVE INFORMATION

SHAPE: SIMILAR SPHERICAL SHAPE
SIDE EDGE SMOOTHNESS: HIGH
MEDICAL HISTORY: YES

**SIMILAR CASE** 450     450

○   ◇   ○

460

490

△ PRIMARY LUNG CANCER
○ SPREAD TO LUNG
◇ BENIGN NODULE

470

DIAGNOSTIC NAME: SPREAD TO LUNG

480

MEDICAL INFORMATION

SHAPE: SIMILAR SPHERICAL SHAPE
CALCIFICATION: NO
SIDE EDGE SMOOTHNESS: HIGH
SPICULA: NO
NOTCH: NO
MEDICAL HISTORY: YES
TUMOR MARKER: ABNORMAL VALUE

400

EP 3 410 441 A1

FIG. 6

TARGET CASE 410 410 ... 400

INFERRD RESULT: PRIMARY LUNG CANCER ... 420

LARGE WEIGHT MEDICAL INFORMATION ... 640

1: SHAPE (LOBULATED SHAPE)
2: SPICULA
    (A PLURALITY OF SPICULAE)
3:
4: SIDE EDGE SMOOTHNESS (HIGH)
5: NOTCH: (YES)

SIMILAR CASE 490 450 450

94.5   87.8   74.2 ... 660

63.3   59.9

DIAGNOSTIC NAME: SPREAD TO LUNG ... 470

MEDICAL INFORMATION ... 480 690

SHAPE: LOBULATED SHAPE
CALCIFICATION: NO
SIDE EDGE SMOOTHNESS: INTERMEDIATE
SPICULA: A PLURALITY OF SPICULAE
NOTCH: YES
MEDICAL HISTORY: NO
TUMOR MARKER: ABNORMAL VALUE

EP 3 410 441 A1

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/114867 A1 (KONDO KENJI [JP] ET AL) 9 May 2013 (2013-05-09) * paragraphs [0090] - [0095], [0207] - [0210], [0227], [0256] * | 1-21 | INV. G16H30/40 G16H50/70 |
| X | US 2013/006087 A1 (KONDO KENJI [JP] ET AL) 3 January 2013 (2013-01-03) * paragraphs [0047], [0143], [0153] * | 1-21 | |
| X | US 2010/226550 A1 (MIYASA KAZUHIRO [JP] ET AL) 9 September 2010 (2010-09-09) * paragraphs [0030], [0128], [0138]; figures 9A, 9B * | 1-21 | |
| X | US 2014/089000 A1 (TAKATA KAZUTOYO [JP] ET AL) 27 March 2014 (2014-03-27) * paragraphs [0037], [0085], [0114], [0118], [0121] * | 1-21 | |
| X | US 2013/044925 A1 (KOZUKA KAZUKI [JP] ET AL) 21 February 2013 (2013-02-21) * paragraphs [0004], [0067], [0107], [0127], [0138] - [0142] * | 1,20,21 | TECHNICAL FIELDS SEARCHED (IPC) G16H |
| X | JP 2012 198846 A (HITACHI MEDICAL CORP) 18 October 2012 (2012-10-18) * paragraphs [0007], [0027] - [0030], [0037] * | 1-21 | |

The present search report has been drawn up for all claims

| Place of search | Date of completion of the search | Examiner |
|---|---|---|
| Munich | 7 September 2018 | Laub, Christoph |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 18 17 5382

This annex lists the patent family members relating to the patent documents cited in the above-mentioned European search report.
The members are as contained in the European Patent Office EDP file on
The European Patent Office is in no way liable for these particulars which are merely given for the purpose of information.

07-09-2018

| Patent document cited in search report | | Publication date | Patent family member(s) | | Publication date |
|---|---|---|---|---|---|
| US 2013114867 | A1 | 09-05-2013 | CN | 103200861 A | 10-07-2013 |
| | | | JP | 5054252 B1 | 24-10-2012 |
| | | | JP | WO2013065090 A1 | 02-04-2015 |
| | | | US | 2013114867 A1 | 09-05-2013 |
| | | | WO | 2013065090 A1 | 10-05-2013 |
| US 2013006087 | A1 | 03-01-2013 | JP | 4979842 B1 | 18-07-2012 |
| | | | JP | WO2013001584 A1 | 23-02-2015 |
| | | | US | 2013006087 A1 | 03-01-2013 |
| US 2010226550 | A1 | 09-09-2010 | JP | 5317716 B2 | 16-10-2013 |
| | | | JP | 2010165127 A | 29-07-2010 |
| | | | US | 2010226550 A1 | 09-09-2010 |
| | | | US | 2012250959 A1 | 04-10-2012 |
| | | | WO | 2010082246 A1 | 22-07-2010 |
| US 2014089000 | A1 | 27-03-2014 | JP | 5462414 B2 | 02-04-2014 |
| | | | JP | WO2013001678 A1 | 23-02-2015 |
| | | | US | 2014089000 A1 | 27-03-2014 |
| | | | WO | 2013001678 A1 | 03-01-2013 |
| US 2013044925 | A1 | 21-02-2013 | CN | 102985924 A | 20-03-2013 |
| | | | JP | 5139603 B2 | 06-02-2013 |
| | | | JP | WO2012111288 A1 | 03-07-2014 |
| | | | US | 2013044925 A1 | 21-02-2013 |
| | | | WO | 2012111288 A1 | 23-08-2012 |
| JP 2012198846 | A | 18-10-2012 | JP | 5739700 B2 | 24-06-2015 |
| | | | JP | 2012198846 A | 18-10-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011118543 A